# EUROPÄISCHE PATENTANMELDUNG

(11) **EP 2 218 400 A1**
(43) Veröffentlichungstag der Anmeldung: **18.08.2010**
(21) Anmeldenummer: 09001937.3
(22) Anmeldetag: 12.02.2009
(51) Int. Cl.: A61B 5/151

(54) **Lanzette mit Testbereich**

(71) Anmelder: Roche Diagnostics GmbH, 68305 Mannheim (DE); F.HOFFMANN-LA ROCHE AG, 4070 Basel (CH)
(72) Erfinder: Konya, Ahmet, 97165 Waldsee (DE); Hörauf, Christian, 68723 Oftersheim (DE)
(74) Vertreter: Twelmeier Mommer & Partner

(57) **Zusammenfassung**

Die Erfindung betrifft eine Lanzette mit einem Grundkörper (2), der auf einer Oberseite einen Testbereich (3) zur Untersuchung einer Körperflüssigkeitsprobe hat, einem von dem Grundkörper (2) ausgehenden Stechelement (4) zum Einstich in die Haut eines Patienten, und einer Kapillarstruktur (5), um eine Körperflüssigkeitsprobe von dem Stechelement (4) zu dem Testbereich (3) zu transportieren. Erfindungsgemäß ist vorgesehen, dass die Kapillarstruktur (5) einen Abschnitt aufweist, der einen Flüssigkeitstransport in einer Richtung bewirkt, die in einer Draufsicht auf die Oberseite des Grundkörpers (2) eine senkrecht zur Längsrichtung des Stechelements (4) verlaufende Komponente hat.

## Beschreibung

Die Erfindung geht aus von einer Lanzette mit den im Oberbegriff des Anspruchs 1 angegebenen Merkmalen wie sie aus der EP 1 360 933 B1 oder der WO 2005/084530 A2 bekannt ist. Eine solche Lanzette hat einen Grundkörper mit einem Testbereich zur Untersuchung einer Körperflüssigkeitsprobe, ein von dem Grundkörper ausgehendes Stechelement zum Einstich in die Haut eines Patienten, das sich in einer Stichrichtung erstreckt, und eine Kapillarstruktur, um eine Körperflüssigkeitsprobe von dem Stechelement zu dem Testbereich zu transportieren. Die Erfindung betrifft ferner ein Stechsystem umfassend ein Stechgerät und derartige Lanzetten.

Lanzetten, die mit dazu passenden Handgeräten ein Stechsystem bilden, werden beispielsweise von Diabetikern benötigt, die ihren Blutzuckerspiegel mehrmals täglich überprüfen müssen und dafür eine durch eine Stichwunde gewonnene Körperflüssigkeitsprobe benötigen. Ständiges Ziel bei der Entwicklung derartiger Stechsysteme ist es, diese so klein und kompakt wie möglich auszubilden. Dies gilt nicht nur für Handgeräte zur Messung einer Analytkonzentration, beispielsweise der Glukosekonzentration oder der Laktatkonzentration, sondern insbesondere auch für die von Handgeräten benötigten Lanzetten. Je kleiner die Lanzetten nämlich sind, desto größer ist der Lanzettenvorrat, der in einem Handgerät mitgeführt werden kann.

Aufgabe der Erfindung ist es deshalb, einen Weg aufzuzeigen, wie ein kompaktes Stechsystem zur Untersuchung einer Körperflüssigkeitsprobe verwirklicht werden kann.

Diese Aufgabe wird durch eine Lanzette mit den im Anspruch 1 angegebenen Merkmalen gelöst. Vorteilhafte Weiterbildungen der Erfindung sind Gegenstand von Unteransprüchen. Die Erfindung wird ferner durch ein Stechsystem mit erfindungsgemäßen Lanzetten und einem Stechgerät gemäß Anspruch 15 gelöst.

In einer Draufsicht auf die Oberseite gesehen sind bei einer erfindungsgemäßen Lanzette das Stechelement und der Testbereich zueinander seitlich versetzt angeordnet und die Kapillarstruktur weist einen Abschnitt auf, der Flüssigkeit seitwärts transportiert.

Um Flüssigkeit seitwärts zu transportieren, kann die Kapillarstruktur beispielsweise einen Abschnitt aufweisen, der in der Draufsicht gesehen quer, also beispielsweise senkrecht oder schräg, zur Längsrichtung des Stechelements verläuft. Der Betreffende Abschnitt kann geradlinig verlaufen, muss es aber nicht. Möglich ist es deshalb auch, dass dieser Abschnitt der Kapillarstruktur einer Draufsicht in einem Bogen verläuft.

Allgemein gesagt kann die Kapillarstruktur einen Abschnitt aufweisen, der einen Flüssigkeitstransport in einer Richtung bewirkt, die in einer Draufsicht auf die Oberseite des Grundkörpers eine senkrecht zur Längsrichtung des Stechelements verlaufende Komponente hat. Die Richtung kann gegebenenfalls weitere Komponenten haben, die Längsrichtung des Stechelements oder senkrecht zur Oberseite verlaufen.

Die erfindungsgemäße Anordnung der Kapillarstruktur ermöglicht es, den Testbereich in Bezug auf die Längsrichtung des Stechelements seitlich versetzt von dem Stechelement anzuordnen, so dass der Testbereich nicht in direkter Linie hinter dem Stechelement sitzt. Bei einer erfindungsgemäßen Lanzette sich deshalb erreichen, dass auch bei einer sehr kleinen Lanzette eine Ankopplung an einen Stechantrieb eines Stechgeräts möglich ist, ohne die Untersuchung einer Körperflüssigkeitsprobe in dem Testbereich zu beieinträchtigen.

Bei einer erfindungsgemäßen Lanzette kann nämlich ein Kopplungsbereich, an dem der Stechantrieb eines Stechgeräts ankoppelt, in gerader Linie hinter dem Stechelement und neben dem Testbereich angeordnet werden. Auf diese Weise kann die Länge der Lanzette ohne Einschränkung ihrer Funktionalität wesentlich verkürzt werden.

Insbesondere kann bei dem erfindungsgemäßen Stechsystem der Stechantrieb eines Stechgeräts in Stichrichtung hinter dem Stechelement an die Lanzette ankoppeln - was Kippmomente und deshalb auch schmerzerzeugende Vibrationen minimiert - und eine geradlinige Stichbewegung bewirken. Indem der Testbereich seitlich neben dem Kopplungsbereich angeordnet ist, bleibt der Testbereich dabei für eine Messeinheit zugänglich. Vorteilhaft kann an dem Testbereich einer erfindungsgemäßen Lanzette eine Messung vorgenommen werden, während die Lanzette an den Stechantrieb angekoppelt ist. Nach einem Stich ist deshalb bei einer erfindungsgemäßen Lanzette keine aufwendige Repositionierung in Bezug auf eine Messeinheit erforderlich. Das Handgerät eines erfindungsgemäßen Stechsystems kann deshalb trotz kleiner Lanzetten und kleiner Probenmengen mit einer relativ einfachen und kostengünstigen Mechanik auskommen.

Eine vorteilhafte Weiterbildung der Erfindung sieht vor, dass die Kapillarstruktur als Kapillarkanal ausgebildet ist, beispielsweise als eine Rinne oder ein Schlitz. Eine solche Rinne oder ein Schlitz können offen sein oder durch eine Abdeckung, beispielsweise eine Folie, bedeckt sein. Bevorzugt hat die Kapillarstruktur eine hydrophile Oberfläche, was sich insbesondere durch eine hydrophile Beschichtung, beispielsweise mit Heparin, erreichen lässt. Unter einer Kapillarstruktur ist dabei eine Struktur zu verstehen, die durch Kapillarkräfte einen Flüssigkeitstransport bewirkt.

Bevorzugt hat der Kapillarkanal einen Knick oder eine Kurve. Besonders bevorzugt ist dabei, dass der Kapillarkanal im Bereich des Knicks oder der Kurve eine Querschnittsfläche aufweist, die höchstens so groß wie in einem angrenzenden Abschnitt ist. Indem in einem Knick die Ecken angerundet werden, lässt sich eine Zunahme des Abstands zwischen gegenüberliegenden Rändern des Kanals im Bereich des Knicks verhindern und somit für eine gleich bleibende Querschnittsfläche sorgen. Die Querschnittsfläche ist dabei jeweils senkrecht zur lokalen Strömungsrichtung zu orientieren.

Eine weitere vorteilhafte Weiterbildung der Erfindung sieht vor, dass Kapillarstruktur einen Flüssigkeitstransport in einer Ebene bewirkt. Geeignet Kapillarstrukturen lassen sich mit vorteilhaft geringem Aufwand ausbilden, insbesondere auf einer ebenen Oberseite des Grundkörpers, beispielsweise durch Ätzen.

Der Grundkörper einer erfindungsgemäßen Lanzette kann einstückig mit dem Stechelement ausgebildet sein, beispielsweise indem die Kontur des Grundkörpers mit dem von ihm ausgehenden Stechelement aus einem Blech herausgeschnitten wird, beispielsweise durch Laserstrahlschneiden, Stanzen oder Ätzen. Möglich ist es aber auch, dass der Grundkörper und das Stechelement separate Bauteile sind, die bei der Herstellung der Lanzette zusammengefügt werden.

Eine weitere vorteilhafte Weiterbildung der Erfindung sieht vor, dass der Testbereich in Bezug auf die Längsrichtung des Stechelements seitlich mindestens um die Breite des Stechelements versetzt angeordnet ist. Der Versatz ist dabei von der Mitte des Stechelements bis zur Mitte des Testbereichs zu messen. Das Stechelement hat dabei bevorzugt in einem an seine Spitze anschließenden Bereich, der bei einem Stich in die Haut eines Patienten eindringt, eine gleich bleibende Breite, kann sich jedoch bis zu dem Grundkörper auch zu nehmend verbreitern. Maßgeblich ist Breite des Stechelements am Ende des bei einem Stich bestimmungsgemäß in die Haut eines Patienten eindringenden Bereichs.

Eine weitere vorteilhafte Weiterbildung der Erfindung sieht vor, dass das Stechelement in einer Draufsicht außermittig an dem Grundkörper angeordnet ist, also von dem linken und dem rechten Rand des Grundkörpers unterschiedlich weit entfernt ist. Auf diese Weise kann eine besonders kompakte Lanzette mit einem seitlich versetzt von dem Stechelement angeordneten Testbereich geschaffen werden. Der Grundkörper kann sich seitlich von dem Stechelement mit einem den Testbereich tragenden Abschnitt weiter als auf der entgegen gesetzten Seite von dem Stechelement erstrecken, beispielsweise um die Hälfte weiter, doppelt so weit oder noch weiter.

Eine weitere vorteilhafte Weiterbildung der Erfindung sieht vor, dass der Testbereich als ein aufgeklebtes Testfeld mit Nachweisreagenzien ausgebildet ist. Entsprechende Testfelder mit Nachweisreagenzien zur fotometrischen oder elektrochemischen Bestimmung einer Analytkonzentration sind bei handelsüblichen Teststreifen, beispielsweise zur Bestimmung der Glukosekonzentration vorhanden und bedürfen deshalb keiner weiteren Erläuterung. Anstelle eines aufgeklebten Testfeldes kann der Testbereich beispielsweise auch als eine Küvette ausgebildet sein, in der eine Körperflüssigkeitsprobe optisch untersucht werden kann, insbesondere reagenzfrei untersucht werden kann.

Weitere Einzelheiten und Vorteile der Erfindung werden an Ausführungsbeispielen unter Bezugnahme auf die beigefügten Zeichnungen erläutert. Gleiche und einander entsprechende Teile sind dabei mit übereinstimmenden Bezugszahlen bezeichnet.

### Es zeigen:

- Fig. 1: eine schematische Darstellung eines Ausführungsbeispiels einer erfindungsgemäßen Lanzette in einer Draufsicht auf eine Oberseite ihres Grundkörpers;
- Fig. 2: eine schematische Darstellung eines weiteren Ausführungsbeispiels einer erfindungsgemäßen Lanzette;
- Fig. 3: eine schematische Darstellung eines weiteren Ausführungsbeispiels einer erfindungsgemäßen Lanzette;
- Fig.4: eine schematische Darstellung eines Lanzettenträgerbands mit mehreren Lanzetten gemäß Figur 1; und
- Fig.5: eine schematische Darstellung eines Ausführungsbeispiels eines Stechsystems.

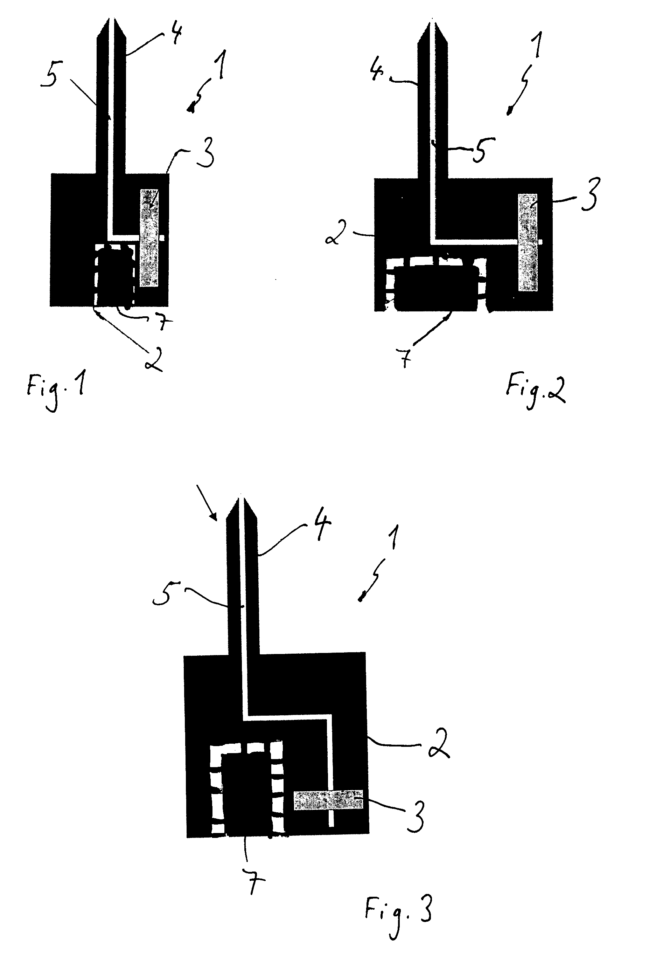

Die in Figur 1 dargestellte Lanzette 1 hat einen Grundkörper 2 mit einem Testbereich 3 zur Untersuchung einer Körperflüssigkeitsprobe, ein von dem Grundkörper 2 ausgehendes Stechelement 4 zum Einstich in die Haut eines Patienten und eine Kapillarstruktur 5, um eine Körperflüssigkeitsprobe von dem Stechelement 4 zu dem Testbereich 3 zu transportieren. Das Stechelement 4 erstreckt sich in einer Längsrichtung, die mit der Stichrichtung übereinstimmt, in der die Lanzette 1 bei Gebrauch eine geradlinige Stichbewegung ausführt.

Das Stechelement 4 ist in Stichrichtung gesehen seitlich versetzt von dem Testbereich 3 angeordnet. Die Kapillarstruktur 5 hat deshalb einen Abschnitt, der einen Flüssigkeitstransport quer zur Stichrichtung bewirkt. Bei dem dargestellten Ausführungsbeispiel ist der quer zur Längsrichtung des Stechelements 4 verlaufende Abschnitt der Kapillarstruktur 5 senkrecht zur Längsrichtung des Stechelements 4 angeordnet. Prinzipiell kann der quer zur Längsrichtung des Stechelements 4 verlaufende Abschnitt der Kapillarstruktur 5 aber auch schräg zur Längsrichtung des Stechelements 4 oder bogenförmig verlaufen.

Der quer zur Stichrichtung verlaufende Abschnitt der Kapillarstruktur 5 ist ebenso wie der Testbereich 3 auf dem Grundkörper 2 angeordnet. Die Kapillarstruktur 5 ist bevorzugt als ein Kapillarkanal ausgebildet, beispielsweise in Form einer Rinne oder eines durchgehenden Schlitzes. Die Kapillarstruktur 5 erstreckt von einem vorderen Bereich des Stechelementes, der bei einem Stich in den Körper eines Patienten eindringt auf den Grundkörper 2 und dort bis zu dem seitlich versetzt angeordneten Testbereich. Bevorzugt ist die Kapillarstruktur 5, wie in Figur 1 dargestellt, zur Spitze hin offen. Möglich ist es aber auch, dass die Kapillarstruktur 4 erst kurz hinter der Spitze in einem Bereich beginnt, der bei einem Stich in den Körper eines Patienten eindringt.

Die Kapillarstruktur 5 verläuft in dem Stechelement 4 und in einem daran anschließenden Bereich des Grundkörpers 2 geradlinig in Stichrichtung. Die Kapillarstruktur 5 hat deshalb einen Knick oder eine Kurve. Durch den gerade in dem Stechelement 4 verlaufenden Anfangsabschnitt der Kapillarstruktur 5 ist eine geometrische Gerade definiert, die in einem Abstand an dem Testbereich 3 vorbei verläuft. Der Testbereich 3 ist nämlich in Bezug auf die Längsrichtung des Stechelements 4 seitlich versetzt von dem Stechelement 4 angeordnet. Auf diese Weise ist in Stichrichtung gesehen seitlich neben dem Testbereich 3 Platz für einen Kopplungsbereich 7 zum Ankoppeln der Lanzette 1 an einen Stechantrieb eines Handgeräts. Der in Figur 1 durch eine durchbrochene Linie angedeutete Kopplungsbereich 7 kann als eine Kopplungsfläche ausgeführt sein, die sich nicht von der übrigen Oberfläche des Grundkörpers 2 unterscheidet, oder mit einer Kopplungsstruktur versehen sein, beispielsweise Rillen oder andere Vertiefungen aufweisen.

Der Kopplungsbereich 7 ist vorteilhaft derart angeordnet ist, dass eine geometrische Gerade, die durch den Anfangsabschnitt der Kapillarstruktur 5 definiert ist, den Kopplungsbereich 7 schneidet. Der Testbereich 3 ist dabei in Bezug auf die Längsrichtung des Stechelements 4 seitlich neben dem Kopplungsbereich 7 angeordnet.

Der Grundkörper 2 der Lanzette 1 ist bevorzugt plattenförmig ausgebildet. Die auf der Oberseite des Grundkörpers 2 ausgebildete Kapillarstruktur 5 ist deshalb bei dem dargestellten Ausführungsbeispiel in einer Ebene angeordnet und bewirkt einen Flüssigkeitstransport in einer Ebene, nämlich auf der im wesentlichen flachen Oberseite des Grundkörpers 2.

Beispielsweise können zur Herstellung der Lanzette 1 die Kontur des Grundkörpers 2 mit dem davon ausgehenden Stechelement 4 aus einem Stahlblech ausgeschnitten werden. Möglich ist es aber auch, den Grundkörper 2 und das Stechelement 4 separat herzustellen und bei der Herstellung der Lanzette 1 miteinander zu verbinden.

Die Figuren 2 und 3 zeigen weitere Ausführungsbeispiele einer Flachlanzette 1. Wie bei dem in Figur 1 dargestellten Ausführungsbeispiel ist das Stechelement 4 in Stichrichtung gesehen seitlich neben dem Testbereich 3 angeordnet. Bei den Ausführungsbeispielen der Figuren 2 und 3 geht das Stechelement 4 jedoch nicht mittig von einer Seite des Grundkörpers 2 aus. Stattdessen ist das Stechelement 4 seitlich versetzt an dem Grundkörper 2 angeordnet.

Bei den in den Figuren 2 und 3 dargestellten Ausführungsbeispielen erstreckt sich der Grundkörper 2 seitlich von dem Stechelement 4 mit seinem den Testbereich 3 tragenden Abschnitt mindestens doppelt so weit wie auf der entgegen gesetzten Seite des Stechelements 4. Bei den Ausführungsbeispielen der Figuren 2 und 3 erstreckt sich der Grundkörper 2 auf der Seite des Stechelements 4, auf welcher der Testbereich 3 angeordnet ist, mehr als doppelt so weit wie auf der anderen Seite des Stechelements 4. Bevorzugt ist dabei, dass der Grundkörper 2 sich auf beiden Seiten seitlich von dem Stechelement 4 erstreckt, wie dies in den Figuren 2 und 3 dargestellt ist. Prinzipiell ist jedoch auch möglich, dass sich der Grundkörper 2 quer zur Stichrichtung nur auf einer Seite des Stechelements 4 erstreckt und auf der anderen Seite bündig mit dem Stechelement 4 endet.

Bevorzugt hat jede Lanzette 1 nur einen einzigen Testbereich 3. Dieser ist bei den dargestellten Ausführungsbeispielen als ein auf eine Oberseite des Grundkörpers aufgeklebtes Testfeld mit Nachweisreagenzien ausgebildet. Die Kapillarstruktur 5 führt bei dem dargestellten Ausführungsbeispiel von dem Stechelement 4 zu dem Testbereich 3 und erstreckt sich über den Testbereich 3 hinaus, so dass der Testbereich 3 eine Körperflüssigkeitsprobe besonders effizient aufnehmen kann. Bevorzugt ist der Testbereich 3, wie in den Figuren dargestellt, streifenförmig ausgebildet.

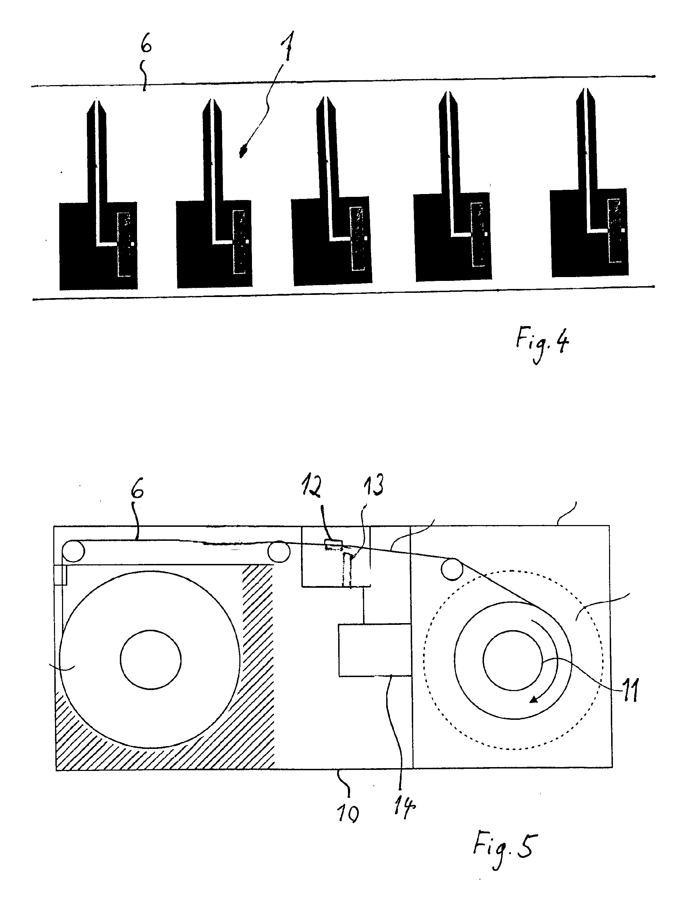

Figur 4 zeigt in einer schematischen Darstellung ein Lanzettenträgerband 6, das mehrere Flachlanzetten 1 trägt, die beispielsweise gemäß Figur 1 ausgebildet sein können. Bei dem dargestellten Ausführungsbeispiel sind die Lanzette 1 quer zur Längsrichtung des Trägerbandes 6, genauer gesagt senkrecht zur Längsrichtung orientiert. Ein solches Lanzettenträgerband 6 kann beispielsweise in einer Bandkassette vertrieben werden, die in ein passendes Aufnahmefach eines Geräts eingesetzt wird.

Figur 5 zeigt eine schematische Darstellung eines Stechsystems, das aus einem Handgerät 10 und Lanzetten 1, wie sie beispielhaft vorstehend beschriebenen wurden, besteht. In dem Stechgerät 10 ist ein Lanzettenträgerband 6 mit Lanzetten 1 angeordnet. Das Handgerät 10 hat eine Wickeleinrichtung 11, um das Trägerband 6 schrittweise aufzuwickeln und so die von dem Trägerband 6 getragenen Lanzetten 1 nacheinander in eine Gebrauchsposition zu bringen, in der sie von einem Stechantrieb 12 in eine geradlinige Stichbewegung versetzt werden können. Der Stechantrieb 12 hat einen Lanzettenhalter, um eine Lanzette 1 zusammen mit einem sie tragenden Trägerbandabschnitt zu klemmen. Bei dem dargestellten Ausführungsbeispiel erfolgt die Stichrichtung senkrecht zur Zeichenebene. Bei einem Stich wird das Lanzettenträgerband 6 umgebogen, so dass sich die Spitze des Stechelements 4 von dem Trägerband 6 abhebt, wie dies aus der WO 2008/083844 A1 bekannt ist.

Das Stechgerät 10 weist ferner eine Messeinheit 13 auf, um eine Körperflüssigkeitsprobe in dem Testbereich 3 zu untersuchen. Da der Testbereich 3 der Lanzetten 1 in Stichrichtung seitlich versetzt hinter dem Stechelement 4 angeordnet ist, lässt der Lanzettenhalter den Testbereich 3 einer gehaltenen Lanzette 1 frei. Der Testbereich 3 und eine von ihm aufgenommene Probe können deshalb von der Messeinheit 13, beispielsweise durch eine photometrische Messung, untersucht werden, während die Lanzette 1 von dem Lanzettenhalter gehalten ist.

Die Messeinheit 13 und der Stechantrieb 12 können mit einer Steuer- und Auswerteeinheit 14 verbunden sein, welche Messungen auswertet und das Anzeigen von Messergebnissen auf einer Anzeigeinrichtung, beispielsweise einer Flüssigkristallanzeige bewirkt.

### Bezugszahlen

- 1: Lanzette
- 2: Grundkörper
- 3: Testbereich
- 4: Stechelement
- 5: Kapillarstruktur
- 6: Lanzettenträgerband
- 7: Kopplungsbereich
- 10: Handgerät
- 11: Wickeleinrichtung
- 12: Stechantrieb
- 13: Messeinheit
- 14: Steuer- und Auswerteeinheit

## Patentansprüche

1. Lanzette mit
einem Grundkörper (2), der auf einer Oberseite einen Testbereich (3) zur Untersuchung einer Körperflüssigkeitsprobe hat,
einem von dem Grundkörper (2) ausgehenden Stechelement (4) zum Einstich in die Haut eines Patienten, und
einer Kapillarstruktur (5), um eine Körperflüssigkeitsprobe von dem Stechelement (4) zu dem Testbereich (3) zu transportieren,
**dadurch gekennzeichnet, dass**
in einer Draufsicht auf die Oberseite gesehen das Stechelement (4) und der Testbereich (3) zueinander seitlich versetzt angeordnet sind und die Kapillarstruktur (5) einen Abschnitt aufweist, der Flüssigkeit seitwärts transportiert.

2. Lanzette nach Anspruch 1, **dadurch gekennzeichnet, dass** der Testbereich (3) in Bezug auf die Längsrichtung des Stechelements (4) seitlich mindestens um die Breite des Stechelements (4) versetzt angeordnet ist.

3. Lanzette nach einem der vorstehenden Ansprüche, **dadurch gekennzeichnet, dass** der Grundkörper (2) plattenförmig ist.

4. Lanzette nach einem der vorstehenden Ansprüche, **dadurch gekennzeichnet, dass** die Kapillarstruktur (5) einen Flüssigkeitstransport in einer Ebene bewirkt.

5. Lanzette nach einem der vorstehenden Ansprüche, **dadurch gekennzeichnet, dass** der Grundköper (2) einstückig mit dem Stechelement (4) ausgebildet ist.

6. Lanzette nach einem der vorstehenden Ansprüche, **dadurch gekennzeichnet, dass** in dem Stechelement (4) ein gerade verlaufender Anfangsabschnitt der Kapillarstruktur (5) angeordnet ist.

7. Lanzette nach Anspruch 6, **dadurch gekennzeichnet, dass** der Testbereich (3) derart angeordnet ist, dass eine geometrische Gerade, die durch den Anfangsabschnitt der Kapillarstruktur (5) definiert ist, in einem Abstand an dem Testbereich (3) vorbei verläuft.

8. Lanzette nach Anspruch 6 oder 7, **dadurch gekennzeichnet, dass** der Grundkörper (2) einen Kopplungsbereich (7) zum Ankoppeln der Lanzette (1) an einen Stechantrieb (12) eines Handgeräts (10) aufweist, wobei der Kopplungsbereich (7) derart angeordnet ist, dass eine geometrische Gerade, die durch den Anfangsabschnitt der Kapillarstruktur (5) definiert ist, den Kopplungsbereich (7) schneidet, und wobei der Testbereich (3) in Bezug auf die Längsrichtung des Stechelements (4) seitlich neben dem Kopplungsbereich (7) angeordnet ist.

9. Lanzette nach einem der vorstehenden Ansprüche, **dadurch gekennzeichnet, dass** das Stechelement (4) in einer Draufsicht außermittig an dem Grundkörper (2) angeordnet ist.

10. Lanzette nach einem der vorstehenden Ansprüche, **dadurch gekennzeichnet, dass** die Lanzette (1) nur einen einzigen Testbereich (3) aufweist.

11. Lanzette nach einem der vorstehenden Ansprüche, **dadurch gekennzeichnet, dass** zumindest ein Abschnitt der Kapillarstruktur (5) als ein Kapillarkanal ausgebildet ist.

12. Lanzette nach Anspruch 11, **dadurch gekennzeichnet, dass** der Kapillarkanal (5) einen Knick oder eine Kurve aufweist.

13. Lanzette nach Anspruch 12, **dadurch gekennzeichnet, dass** der Kapillarkanal (5) im Bereich des Knicks oder der Kurve eine Querschnittsfläche aufweist, die höchstens so groß wie in einem angrenzenden Abschnitt ist.

14. Lanzettenträgerband mit mehreren Lanzetten (1) nach einem der vorstehenden Ansprüche.

15. Stechsystem mit
Lanzetten (1) nach einem der vorstehenden Ansprüche und
einem Stechgerät (10), das einen Stechantrieb (12) aufweist, um die Lanzetten (1) in eine geradlinige Stichbewegung zu versetzen, und eine Messeinheit (13) aufweist, um eine Körperflüssigkeitsprobe in dem Testbereich (3) zu untersuchen.
